(19) Europäisches Patentamt

European Patent Office

Office européen des brevets

(11) Publication number: **0 314 206 B1**

(12) **EUROPEAN PATENT SPECIFICATION**

(45) Date of publication of patent specification: **27.10.93**   (51) Int. Cl.⁵: **A61K 9/22**, A61M 31/00

(21) Application number: **88202033.2**

(22) Date of filing: **16.09.88**

(54) **Solubility modulated drug delivery device.**

(30) Priority: **24.09.87 US 100664**
**02.11.87 US 115487**

(43) Date of publication of application:
**03.05.89 Bulletin 89/18**

(45) Publication of the grant of the patent:
**27.10.93 Bulletin 93/43**

(84) Designated Contracting States:
**AT BE CH DE ES FR GB GR IT LI LU NL SE**

(56) References cited:
**EP-A- 0 190 969**
**EP-A- 0 302 693**
**EP-A- 0 309 051**
**US-A- 4 256 108**
**US-A- 4 687 660**

(73) Proprietor: **MERCK & CO. INC.**
**126, East Lincoln Avenue**
**P.O. Box 2000**
**Rahway New Jersey 07065-0900(US)**

(72) Inventor: **McClelland, Gregory A.**
**12830 West 88th Circle No. 75**
**Lenexa Kansas 66215(US)**
Inventor: **Zentner, Gaylen M.**
**2628 Bardith Court**
**Lawrence Kansas 66044(US)**

(74) Representative: **Cole, William Gwyn**
**European Patent Department,**
**Merck & Co., Inc.,**
**Terlings Park,**
**Eastwick Road**
**Harlow, Essex CM20 2OR (GB)**

**Description**

FIELD OF THE INVENTION

This invention pertains to both a useful and novel drug-delivery device for dispensing a drug to an environment of use. Particularly, the invention pertains to an osmotic drug-delivery device containing a controlled release drug solubility modulator that regulates the solubility of the drug(s) within the device. This regulation affects the release profile of the drug from the device. Consequently, selecting the proper drug solubility modulator allows the release of drug to be controlled by the delivery device and not by the intrinsic water solubility of the drug or the environment surrounding the device.

The solubility of drug(s) within the drug-delivery device core, and the attendant drug release pattern from the device, can be regulated in a variety of ways. For example, the solubility of many drugs is dependent upon the pH of the dissolution media. For a basic drug that is slightly soluble at pH 7, adjusting the pH of the dissolution medium into the acid range would result in an increased drug solubility. Similarly, the solubility of acidic drugs is often increased as the pH increases. Thus, preselection and control of the pH of the drug's dissolution medium within the core compartment with controlled-release acid/base solubility modulators permits control of both the drug solubility and release profile. The solubility of many drugs is sensitive to the presence of added electrolytes (salts) which contribute to common ion, salting-in/salting-out, colloidal stability, and other solution effects. When the solubility of the drug is decreased, the effect can be generically referred to as salting-out; if the drug solubility is increased it can be referred to as salting-in. For example, sodium chloride commonly decreases the solubility (salts-out) of many drugs. Preferentially selecting and controlling the electrolyte environment within the device core compartment with controlled-release salt solubility modulators permits the solubility and the release profile of the drug to be controlled. Other means by which the core compartment media of the drug can be controlled and thus regulate the solubility of the drug include but are not limited to: complexation effects; surfactant effects; dielectric effects; polarity effects; solvation effects.

The intent of the present invention is to incorporate the aforementioned solubility modulating effects into the osmotic pump device configuration to control the release profile of the drug from the drug-delivery system. Specifically, this approach permits successful delivery of drugs with inappropriate or non-optimal solubilities from an osmotic pump device. The device thereby provides the desired drug availability for absorption.

BACKGROUND OF THE INVENTION

The need for systems that can deliver a drug at a controlled rate of release to an environment of use over a specified period of time is well established.

Devices for the controlled and continuous delivery of an active agent made from microporous materials are known to the prior art. Generally, the agent is embedded in or surrounded by the material and its release therefrom often is adversely influenced by external conditions. For example, U.S. Pat. No. 2,846,057 discloses a device consisting of a porous cellophane wall surrounding sodium fluoride that is released by water flowing into the pores to dissolve and leach it from the device. Controlled release is hard to obtain with this device because release is governed by external conditions and not by the device. That is, the amount of fluoride released changes with the rate of flow of water, with higher rates increasing the amount released, and lower rates decreasing the amount released over time. Similarly, U.S. Pat. No. 3,538,214 discloses a device consisting of drug coated with a film of water-insoluble plastic containing a modifying agent that is soluble at a certain pH. When this device is in the gastrointestinal tract, the modifying agent is partially or fully dissolved from the film by gastrointestinal fluid to form a porous film. This lets fluid through the film to dissolve the drug and leach it outwards through the pores into the tract. Controlled release is difficult to achieve with this device because the selection of the modifying agent is based on the unknown acid and alkaline state of the gastrointestinal tract which concomitantly influences pore formation and the exposure of drug to fluid. A similar device is disclosed in U.S. Pat. No. 2,928,770. The device of this patent consists of an outer layer of drug coated onto a porous material having its pores filled with a softened wax that is supposedly removed in the gastrointestinal tract by the alimentary fluid. This device cannot be relied on for controlled release because it too requires in situ pore formation which is dominated by unregulated external conditions and not by the device. The use of pore formers in substantially water impermeable polymers is disclosed in J. Pharm. Sci. 72, p. 772-775 and U.S. Patents 4,557,925; 4,244,941; 4,217,878; 3,993,072. These devices release the core components by simple diffusion only and would be subject to environmental agitation.

U.S. Patent 3,957,523 discloses a device which has a pH sensitive pore former in the device wall. U.S. Patents 4,309,996; 4,320,759; 4,235,236 disclose devices with a microporous coat containing a swelling polymer as the driving force for delivery of agents.

U.S. Patents 3,854,770 and 3,916,899 disclose devices which have semipermeable walls that are permeable to water and substantially impermeable to dissolved drugs and solutes. A passageway through the semipermeable wall, disclosed as a drilled hole, is provided as an exit portal for the drug through the wall. U.S. Patents 4,256,108; 4,160,452; 4,200,098 and 4,285,987 disclose devices which contain multiple wall layers, at least one of said walls having a drilled hole for the release of core components through a rate-controlling semipermeable membrane that is substantially impermeable to dissolved drugs and other solutes. The use of solubility modulators that regulate the solubility of the drug(s) within the device to control drug release from the osmotic drug-delivery device were not disclosed. U.S. Patent 4,326,525 is also based on semipermeable membrane technology with a drilled hole acting as exit portal for the drug. This patent discloses the use of buffers which react via proton-transfer or neutralizing reactions with the drug to produce a new drug agent which has different thermodynamic properties from the parent drug.

European patent application no. 0 190 969 describes a drug delivery system which relies upon the ability of a hydrophobic substance to coat the inside wall of a controlled release device such that water soluble, hydrophilic materials such as osmogents are retained within the device until lipid soluble materials have been expelled via osmotic pressure. The osmogents are intimately mixed with the other core components.

European patent application no. 0 309 051 discloses a drug delivery device comprising a core containing a water insoluble, non-diffusable, charged resin, intimately mixed with a water soluble, diffusable, ionized drug surrounded by a water insoluble, semipermeable wall which contains holes that allow release of the drug.

The usefulness of the above devices would be increased if a device and method were provided to improve the delivery of drugs which have been found to be difficult to incorporate into an osmotic drug delivery module without conversion of the parent drug into a new drug whose stability and toxicology are uncharacterized. Further utility results from methodology which provides for a sustaining of the improvement inducing effect through technology which substantially extends the lifetime of the modulating agent(s).

## DESCRIPTION OF DRAWINGS

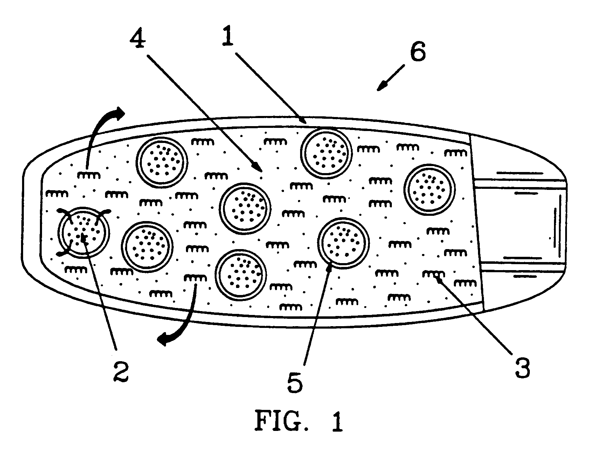

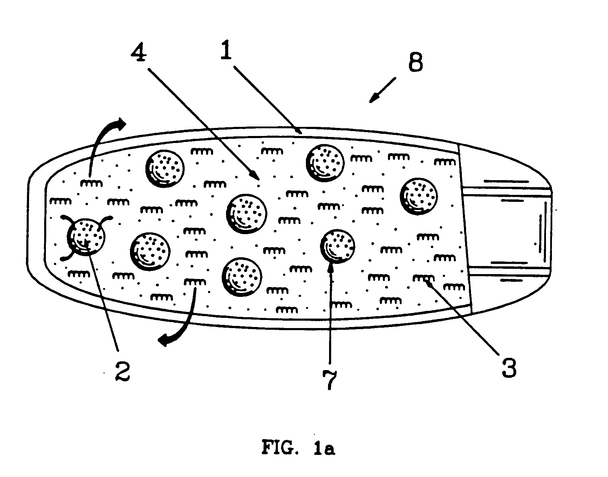

Fig. 1 is a schematic representation of one embodiment of the instant invention. The device, 6, has a core composition comprised of drug(s), 3, solubility modulator(s), 2, surrounded by a rate modifying coat, 5, to form controlled release solubility modulating units that are dispersed among other excipients, 4, which may optionally contain elements found in 2, as needed to form a tablet suitable for the application of a microporous, rate-determining, water-insoluble wall, 1. In operation water permeates wall 1 at a rate controlled by the nature of wall 1, entering the core compartment where water soluble drug and excipients dissolve. The water then permeates coating 5 and the solubility modulator(s), 2, are metered through the coating, 5, into the core environment for a prolonged period where the solubility of drug, 3, is modified. A priming bolus of agent 2 may be provided in 4, to modulate the drug solubility during the lag-time for water and solution to permeate coat 5. Drug, 3, and those excipient and solubility modifiers which are dissolved in the core fluids are then freely permeable to exit the core compartment through wall 1 in response to osmotic and concentration gradients. It is often desirable for the lifetime of agent, 2, and drug, 3, to closely coincide to allow for solubility control throughout the entire delivery period of the drug. However, it is not a necessary requirement that the lifetimes of 2 and 3 be similar; in practice, lifetimes may be adjusted to achieve the kinetic profile of drug release best suited to the therapeutic need. Another embodiment of the present invention is schematically shown as device 8 in Fig. 1a. In this configuration the solubility modulator, 2, is dispersed throughout a matrix, 7, which acts as a carrier for the solubility modulator compound, 2. The solubility modulator, 2, is released from the matrix, 7, by mechanisms of dissolution, diffusion, partitioning, osmosis, or combinations thereof. Elements 1 through 4 were described previously.

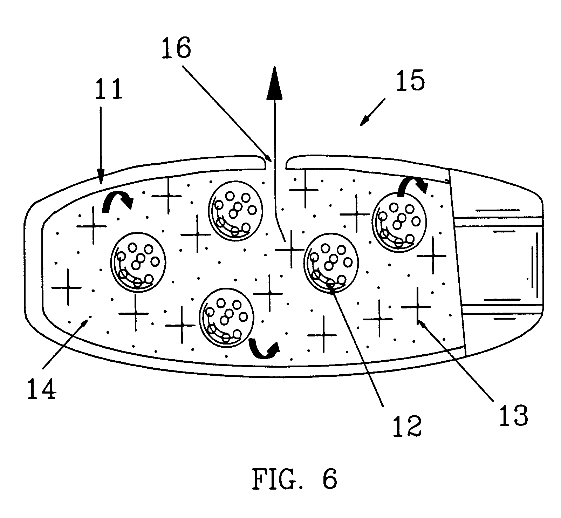

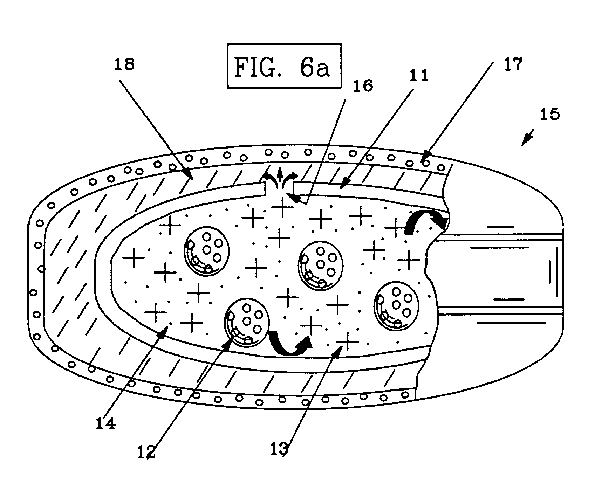

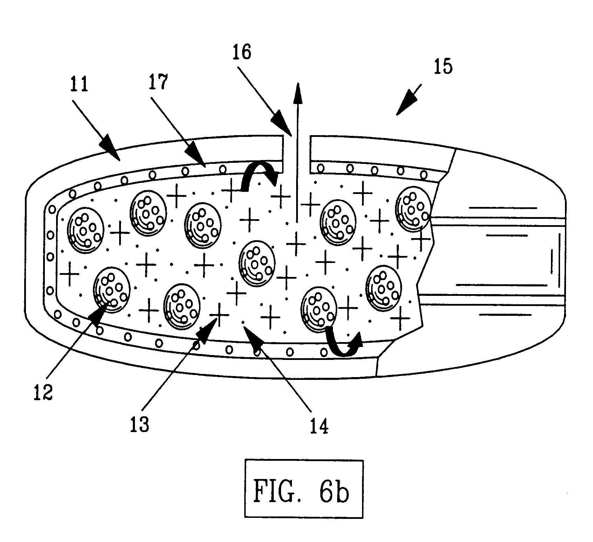

Fig. 6 is a schematic representation of one embodiment of the instant invention. The device, 15, has a core composition comprised of drug(s), 13, solubility modulator(s), 12, surrounded by a rate modifying coat or dispersed throughout a matrix to form controlled release solubility modulating units that are dispersed among other excipients, 14, which may optionally contain elements found in 12, as needed to form a tablet suitable for the application of a semipermeable, rate-determining, water-insoluble wall, 11. In operation water permeates wall 11 at a rate controlled by the nature of wall 11, entering the core compartment where water soluble drug and excipients dissolve. The solubility modulator(s), 12, are metered through the rate-modifying coating or matrix into the core environment for a prolonged period where the solubility of drug, 13, is modified. A priming bolus of agent 12 may be provided in 14, to modulate the drug solubility during the lag-time for water and solution to actuate release of 12. Drug, 13, and those excipients and solubility modifiers which are dissolved in the core fluids are then freely permeable to exit the core compartment through the release means in the wall 11, exemplified here as a hole, 16, in response to osmotic and concentration gradients. It is often desirable for the lifetime of agent, 12, and drug, 13, to closely coincide to allow for solubility control throughout the entire delivery period of the drug. However, it is not a necessary requirement that the lifetimes of 12 and 13 be similar; in practice, lifetimes may be adjusted to achieve the kinetic profile of drug release best suited to the therapeutic need.

Fig. 6a is another embodiment of the instant invention where the semipermeable wall, 11, is coated with a layer of material, 18, that is soluble in fluids of the intended environment of use (commonly water), with a microporous wall, 17, separating the layer, 18, from the external environment. The compound(s) of layer, 18, dissolve and then freely permeate the microporous wall, 17, in a fluid environment, creating a fluid filled zone separating the microporous and semipermeable walls. Drug laden solution that is pumped through the hole, 16, at a rate controlled by the semipermeable wall, 11, enters the now fluid layer, 18, where it may then freely permeate the microporous wall, 17, to the exterior. All other components were defined previously.

Fig. 6b is another embodiment of the instant invention. As configured, the drug containing core is coated with a laminate structure comprised of a microporous wall, 17, immediately contacting the core, and an overcoating semipermeable wall, 11. The microporous wall serves as a base coating to lend mechanical strength and support to the rate controlling semi-permeable wall. A hole, 16, is provided as an exit portal for the drug solution. Other components were defined previously.

## OBJECT OF THE INVENTION

It is an immediate object of this invention to provide a novel device for delivering an agent (drug) to produce a beneficial effect which overcomes the disadvantages associated with prior art devices.

Another object of the invention is to provide a device for delivering an agent at controlled rate over a specified period of time, which delivery is controlled by the device and not the environment surrounding the device.

Another object of the invention is to provide a device for controlled delivery of a drug and a solubility modulating agent where the solubililty, and thus delivery profile, of said drug is controlled by the drug-delivery device and not by the intrinsic water solubility of the drug.

Another object of the invention is to provide a method for converting unacceptable drug release profiles into profiles that have been recognized as therapeutically desirable. For example, drugs with intrinsic water solubilities that are very low will release from osmotic devices at slow rates that may be subtherapeutic; modulation to increase the solubility of such drugs will increase the release rate into the therapeutic range. Conversely, drugs with excessively high intrinsic water solubilities will be released rapidly in a pattern exhibiting a low percentage of constant (zero-order) release; modulation to decrease the solubility of such drugs will increase the percentage of therapeutically desirable zero-order release. The above effects are achieved without chemical modification of the parent drug with attendant stability and toxicological concerns.

Another object of the invention is to provide a drug delivery device that is readily manufacturable to deliver a pre-determined dose of agent at a programmed rate from compositions of matter in the varied geometries and sizes of tablets, pellets, multi-particulates, and such related dosage forms as familiar to those skilled in the art of oral, buccal, vaginal, rectal, nasal, ocular, aural, parenteral, and related routes of administration.

Another object of the invention is to provide a drug delivery device for delivering an active agent over a range of release rates as controlled by the device.

Other objects, features and advantages of the invention will be apparent to those skilled in the art from the following detailed description of the invention, taken in conjunction with the drawings and accompanying claims.

## BRIEF DESCRIPTION OF THE INVENTION

A device is disclosed for delivery of a water soluble beneficial agent. The beneficial agent, commonly a drug, is delivered by osmotic pumping of dissolved drug, and excipients as required, at a controlled rate for a specified period to the environment surrounding the device. The solubility of the beneficial agent is controlled through the influence of a controlled release solubility modulator contained within the drug delivery device. The controlled release solubility modulator influences the release pattern of the beneficial agent. The device is comprised of 1) at least one water soluble beneficial agent and 2) a controlled release solubility modulator such as a water soluble salt, a surfactant or a complexing agent which may be selected to increase or decrease the drug solubility. The controlled release solubility modulator is surrounded by a water insoluble coating containing pore forming additives dispersed throughout said coating.

Components 1) and 2) may be combined with excipients, binders, lubricants, glidants, and bulking agents as needed to form a core compartment of the device. The core is surrounded by a water insoluble wall containing pore forming additive(s) dispersed throughout said wall or a substantially imperforate wall with a release means. In operation water is imbibed into the core compartment. As water enters the core it is further imbibed into the compartment(s) containing the controlled release solubility modulator. The contents of the solubility modulator compartment(s) are delivered into the surrounding environment where they modulate the solubility of the beneficial agent, thereby controlling the release of the beneficial agent from the device. By adjusting the amount and/or type of solubility modulator, the amount and/or type of coating applied to the solubility modulator and the amount and/or type of coating applied to the core compartment, the release profile of the beneficial agent from the core compartment of the device can be adjusted to meet the desired kinetic profile.

DETAILED DESCRIPTION OF THE INVENTION

The instant invention is directed to a drug-delivery device for the controlled release of a therapeutically active ingredient into an environment of use which comprises:

(A) a core composition comprising

(a) a plurality of controlled release solubility modulating units which modulate and increase solubility of said therapeutically active ingredient within said core comprising solubility modulating agents each of which is either a water soluble salt, a complexing agent or a surfactant and which is surrounded by a water insoluble coat containing at least one pore forming additive dispersed throughout said coat;

(b) a therapeutically active ingredient; and

(B) a water insoluble microporous wall surrounding said core composition and prepared from

(i) a polymer material that is permeable to water but substantially impermeable to solute and

(ii) 0.1 to 75% by weight, based on the total weight of (i) and (ii), of at least one water leachable pore forming additive dispersed throughout said wall.

Components (a) and (b) may be combined with excipients, binders, lubricants, glidants, and bulking agents as needed to form a core that is surrounded by a water insoluble, rate-determining wall containing at least one pore forming additive dispersed throughout said wall or a subsantially imperforate semipermeable wall with a means for release of the water diffusible agent.

Other walls, such as microporous walls, and soluble layers that are freely permeable to dissolved solutes may be incorporated in conjunction with the substantially imperforate semipermeable wall.

The term solubility modulating agent as described herein broadly encompasses any water soluble compound that can exert an effect on the solubility of the drug being delivered from the device. Among the groups of compounds that can exert this effect are water soluble salts such as sodium chloride, potassium chloride, ammonium phosphate, calcium chloride, sodium malate, sodium carbonate, sodium acetate and other organic and inorganic salts. Complexation is another method for solubility modulation. Complexes may be classified as metal ion complexes, organic molecular complexes, and occlusion compounds. Specific examples of complexing agents include but are not limited to cyclodextrins, polyethylene glycols, polyvinyl-pyrrolidone, sodium carboxymethylcellulose, tetracycline derivatives, caffeine, picric acid, quinhydrone, hydroquinone, and bile salts and acids. Another group of solubility modulating agents are surfactants. Examples of surfactants include potassium laurate, sodium dodecyl sulfate, hexadecylsulphonic acid, sodium dioctysulphosuccinate, hexa-decyl(cetyl) trimethyl-ammonium bromide, dodecyl-pyridinium chloride, dodecylamine hydrochloride, N-dodecyl-N,N-dimethyl betaine bile acids and salts, acacia, tragacanth, Igepal(™), sorbitan esters (Spans), polysorbates (Tweens), Triton-X(™) analogs, Brij(™) analogs, Myrj(™) analogs, pluronics, tetronics, surface active drug agents such as phenothiazines and tricyclic antidepressants.

The solubility modulating agent can be surrounded by a water insoluble, permeable, coat that contains at least one pore forming additive dispersed throughout said coat. This coat is often applied to the solubility modulating agent by spray-coating procedures. A portion of the solubility modulating agent may be left uncoated to effect immediate availability during the period intervening the onset of release from the controlled release solubility modulating element(s).

5

Other excipients may also be combined with the drug and solubility modulating agent(s) as needed to maintain pH, promote stability, facilitate manufacturability, and provide osmotic activity to the dissolved core compartment solution to effect a desirable release profile. The entire composite is compressed or formed into tablets, beads or multi-particulates, by conventional methodology to form cores onto which a water insoluble wall containing leachable pore forming additives is applied. Thus, the finished device may contain: a) coated solubility modulator; and either b) immediate availability solubility modulator, or mixtures of a) and b), within the core compartment which is then surrounded by a porous wall or an imperforate semipermeable wall with a release means.

One type of rate controlling wall or coating is comprised of (a) polymeric materials that are insoluble in the fluids of the environment of intended use (usually agueous), (b) other added excipients that will dissolve in the environmental fluids or leach out of the walls. The leached walls are sponge-like structures composed of numerous open and closed cells that form a discontinuous interwoven network of void spaces when viewed with a scanning electron microscope. These controlled porosity walls serve as both water entry and core composition solution exit sites. The walls are permeable to both water and solutes, and as constituted in the environment of use have a small solute reflection coefficient, $\sigma$ and display poor semipermeable characteristics when placed in a standard osmosis cell.

The specifications for the wall are summarized below and include:

1. Fluid Permeability of the wall: $6.96 \times 10^{-18}$ to $6.96 \times 10^{-14}$ cm$^3$ sec/g (equivalent to $10^{-5}$ to $10^{-1}$ cm$^3$ mil/cm$^2$ hr atm).

2. Reflection Coefficient: Microporous coats to have a reflection coefficient, $\sigma$ defined as:

$$\sigma = \frac{\text{osmotic volume flux}}{\text{osmotic pressure difference}} \times \frac{\text{hydrostatic pressure difference}}{\text{hydrostatic volume flux}}$$

where $\sigma$ is less than 1, usually 0 to 0.8.

Additional, preferred specifications for the wall include:

1. Plasticizers and Flux Regulating Additives: 0 to 50, preferably 0.001 to 50, parts per 100 parts wall material.

2. Surfactant Additives: 0 to 40, preferably .001 to 40, parts per 100 parts wall material.

3. Wall Thickness: 1 to 1,000, preferably 20 to 500, $\mu$m typically although thinner and thicker fall within the invention.

4. Microporous Nature: 5% to 95% pores between $10 \times 10^{-10}$ metres (10 angstroms) and 100 $\mu$m diameter.

5. Pore Forming Additives: 0.1 to 75%, preferably 0.1 to 50%, by weight, based on the total weight of pore forming additive and polymer, pore forming additive, preferably: a) 0.1 to 50%, preferably 0.1 to 40%, by weight solid additive; b) 0.1 to 40% by weight liquid additive, but no more than 75% total pore formers.

This type of microporous rate controlling water insoluble wall of the instant invention must not be covered on its inner or outer surface by a layer of material that is impermeable to dissolved solutes within the core during the period of operation.

A second type of rate controlling wall of the invention is a material that is semi-permeable, can form films, and does not adversely affect the drug, animal body, or host, for example, a material that is permeable to an external fluid such as water and the like while essentially impermeable to a selected product, drugs, modulating agents, or to other compounds in the device. The selectively-permeable material or membrane forming the wall is insoluble in body fluids and non-erodible or it can be bioerodible after a predetermined period with bioerosion corresponding to the end of the active drug release period. In each instance it is semipermeable to solvent but not to solute(s) and is suitable for construction of the osmotic powered device. Typical materials for forming the wall include membranes known to the art as osmosis and reverse osmosis membranes. Generally, membranes having a fluid permeability of 0.01 to 10 cm$^3$/cm$^2$ per hour or day or higher, at atmospheric pressure against a saturated product solution or saturated solute solution at the temperature of use while simultaneously possessing a high degree of impermeability to the product or solute are useful for manufacturing the devices of the invention. Of course, other semipermeable membranes operable for the purposes of the invention can also be used within the spirit of the invention.

Additional, preferred specifications for the semipermeable wall include:

1. Plasticizers and Flux Regulating Additives: 0 to 50, preferably 0.001 to 50, parts per 100 parts wall material.

2. Surfactant Additives: 0 to 40, preferably .001 to 40, parts per 100 parts wall material.

3. Wall Thickness: 1 to 1,000, preferably 20 to 500, $\mu$m typically, although thinner and thicker fall within the invention.

Any polymer permeable to water but impermeable to solutes as previously defined may be used. Examples include cellulose acetate having a degree of substitution, D.S., meaning the average number of hydroxyl groups on the anhydroglucose unit of the polymer replaced by a substituting group, up to 1 and acetyl content up to 21%; cellulose diacetate having a D.S. of 1 to 2 and an acetyl content of 21 to 35%; cellulose triacetate having a D.S. of 2 to 3 and an acetyl content of 35 and 44.8%; cellulose propionate having an acetyl content of 1.5 to 7% and a propionyl content of 2.5 to 3% and an average combined propionyl content of 39.2 to 45% and a hydroxyl content of 2.8 to 5.4%; cellulose acetate butyrate having a D.S. of 1.8, an acetyl content of 13 to 15%, and a butyryl content of 34 to 39%; cellulose acetate having an acetyl content of 2 to 29.5%, a butyryl content of 17 to 53%, and a hydroxyl content of 0.5 to 4.7%; cellulose triacylates having a D.S. of 2.9 to 3 such as cellulose trivalerate, cellulose trilaurate, cellulose tripalmitate, cellulose trisuccinate, cellulose triheptylate, cellulose tricaprylate, cellulose trioctanoate, and cellulose tripropionate; cellulose diesters having a lower degree of substitution and prepared by the hydrolysis of the corresponding triester to yield cellulose diacylates having a D.S. of 2.2 to 2.6 such as cellulose dicaprylate and cellulose dipentanate; and esters prepared from acyl anhydrides or acyl acids in an esterification reaction to yield esters containing different acyl groups attached to the same cellulose polymer such as cellulose acetate valerate, cellulose acetate succinate, cellulose propionate succinate, cellulose acetate octanoate, cellulose valerate palmitate, cellulose acetate palmitate and cellulose acetate heptanoate.

Additional polymers that can be used for the purpose of the invention include cellulose acetate acetoacetate, cellulose acetate chloroacetate, cellulose acetate furoate, dimethoxyethyl cellulose acetate, cellulose acetate carboxymethoxypropionate, cellulose acetate benzoate, cellulose butyrate naphthylate, cellulose acetate benzoate, methylcellulose acetate, methylcyanoethyl cellulose, cellulose acetate methoxyacetate, cellulose acetate ethoxyacetate, cellulose acetate dimethylsulfamate, ethylcellulose, ethylcellulose dimethylsulfamate, cellulose acetate p-toluene sulfonate, cellulose acetate methylsulfonate, cellulose acetate dipropylsulfamate, cellulose acetate butylsulfonate, cellulose acetate laurate, cellulose stearate, cellulose acetate methylcarbamate, agar acetate, amylose triacetate, beta glucan acetate, beta glucan triacetate, acetaldehyde dimethyl acetate, cellulose acetate ethyl carbamate, cellulose acetate phthalate, cellulose acetate dimethyl aminoacetate, cellulose acetate ethyl carbonate, poly (vinyl methyl) ether copolymers, cellulose acetate with acetylated hydroxyethyl cellulose, hydroxylated ethylenevinylacetate, poly(ortho ester)s, polyacetals, semipermeable polyglycolic or polylactic acid and derivatives thereof, film forming materials with a water sorption of one to fifty percent by weight at ambient temperatures with a presently preferred water sorption of less than thirty percent, acylated polysaccharides, acylated starches, aromatic nitrogen containing polymeric materials that exhibit permeability to aqueous fluids, membranes made from polymeric epoxides, copolymers of alkylene oxides and alkyl glycidyl ethers, polyurethanes, polyacrylate and polymethacrylate polymers, and derivatives and the like. Admixtures of various polymers may also be used.

The polymers described are known to the art or they can be prepared according to the procedures in Encyclopedia of Polymer Science and Technology, Vol. 3, pages 325 to 354, and 459 to 549, published by Interscience Publishers, Inc., New York, in Handbook of Common Polymers by Scott, J. R. and Roff, W. J., 1971, published by CRC Press, Cleveland, Ohio; and in U.S. Pat. Nos. 3,133,132; 3,173,876; 3,276,586; 3,541,055; 3,541,006; and 3,546,142.

The expression "release means" or hole(s) as used herein is comprised of those means and methods suitable for osmotically releasing the drug from the core through the semipermeable wall. The expression includes the following: an aperture, orifice, bore, porous element through which product can migrate, hollow cellulose acetate fibers suitable for passing the drug, capillary tubes and, cracks. The expression also includes bioerodible materials that erode in the environment of use to produce an osmotic passageway of precontrolled dimensions. Typical bioerodible materials suitable for forming a passageway include erodible poly(glycolic) acid and poly(lactic) acid fibers, poly(ortho esters), erodible gelatinous filaments and, poly-(vinyl alcohol),.

Water insoluble, permeable, non-rate controlling microporous walls may be applied to core composition masses prior to the application of the semipermeable wall or subsequent thereto by spray coating procedures. The microporous wall may either directly contact the semipermeable wall to form a bilaminate structure, or, the microporous wall may be separated from the semipermeable wall by a layer of fluid soluble material, which may optionally contain drug, which dissolves in the environment of use, creating a fluid layer separating the microporous and semipermeable walls.

The layer of fluid soluble material which may be positioned between a semipermeable wall containing a hole(s) and a microporous wall, comprises a layer of material selected from organic or inorganic compounds that are soluble in the fluid of the environment of use; drug may optionally be included. Fluid entering the system (commonly water) dissolves the layer to form a solution which is released to the exterior through the microporous wall. Drug laden solution exiting the hole(s) in the semipermeable wall enters this fluid layer at a rate controlled by the semipermeable wall from where the drug is released to the exterior through the microporous wall. Representative inorganic compounds that can be used for forming the layer include magnesium chloride, sodium chloride, lithium chloride, potassium chloride, sodium carbonate, potassium carbonate, manganese carbonate, sodium sulfite, potassium sulfite, calcium bicarbonate, sodium bicarbonate, potassium bicarbonate, sodium sulfate, potassium sulfate, lithium sulfate, magnesium sulfate, potassium acid phosphate, sodium acid phosphate, and the like. Typical organic compounds include carbohydrates such as glucose, sucrose, fructose, raffinose and lactose, and other organic compounds soluble in water and biological fluids such as sorbitol, mannitol, inositol, urea, tartaric acid and tromethamine.

This non-rate controlling microporous wall is comprised of (a) polymeric material that is insoluble in the fluids of the environment of intended use (usually water), (b) other added excipients that will dissolve in the environmental fluids or leach out of the wall. The leached wall is a sponge-like structure composed of numerous open and closed cells that form a discontinuous interwoven network of void spaces when viewed with a scanning electron microscope. The wall is permeable to both water and solutes, and as constituted in the environment of use has a small solute reflection coefficient, $\sigma$ and displays poor semipermeable characteristics when placed in a standard osmosis cell. Additional specifications for the microporous wall include:

1. Wall Thickness: 1 to 1,000, preferably 20 to 500, $\mu$m typically although thinner and thicker fall within the invention.

2. Pore Forming Additives: 0.1 to 75%, by weight, based on the total weight of pore forming additive and polymer, pore forming additive, preferably: a) 0.1 to 50% by weight solid additive; b) 0.1 to 40% by weight liquid additive.

A controlled porosity wall which can be either rate controlling or non-rate contolling can be generically described as having a sponge-like appearance. The pores can be continuous pores that have an opening on both faces of a microporous lamina, pores inter-connected through tortuous paths of regular and irregular shapes including curved, curved-linear, randomly oriented continuous pores, hindered connected pores and other porous paths discernible by microscopic examination. Generally, microporous lamina are defined by the pore size, the number of pores, the tortuosity of the microporous path and the porosity which relates to the size and number of pores. The pore size of a microporous lamina is easily ascertained by measuring the observed pore diameter at the surface of the material under the electron microscope. Generally, materials possessing from 5% to 95% pores and having a pore size of from $10^{-10}$ m (10 angstroms) to 100 $\mu$m can be used.

Pore forming additives may be used in the instant invention. The microporous wall may be formed in situ, by a pore-former being removed by dissolving or leaching it to form the microporous wall during the operation of the system. The pores may also be formed in the wall prior to operation of the system by gas formation within curing polymer solutions which result in voids and pores in the final form of the wall. The pore-former can be a solid or a liquid. The term liquid, for this invention embraces semi-solids, and viscous fluids. The pore-formers can be inorganic or organic. The pore-formers suitable for the invention include pore-formers that can be extracted without any chemical change in the polymer. Solid additives include alkali metal salts such as sodium chloride, sodium bromide, potassium chloride, potassium sulfate, potassium phosphate, sodium benzoate, sodium acetate, sodium citrate and potassium nitrate; the alkaline earth metal salts such as calcium chloride, calcium nitrate, and the like; the transition metal salts such as ferric chloride, ferrous sulfate, zinc sulfate and, cupric chloride. Water may be used as the pore-former. The pore-formers include organic compounds such as dimethyl sulfone, nicotinamide, saccharides and amino acids. The saccharides include the sugars sucrose, glucose, fructose, mannose, galactose, aldohexose, altrose, talose, lactose, monosaccharides, disaccharides, and water soluble polysaccharides. Also, sorbitol, pentaerythritol, mannitol, organic aliphatic and aromatic ols, including diols and polyols, as exemplified by polyhydric alcohols, poly(alkylene glycols), polyglycols, alkylene glycols, poly($\alpha,\omega$)alkylenediols esters or alkylene glycols, polyvinylalcohol, poly vinyl pyrrolidone, and water soluble polymeric materials. Pores may also be formed in the wall by the volatilization of components in a polymer solution or by chemical reactions in a polymer solution which evolves gases prior to application or during application of the solution to the core mass resulting in the creation of polymer foams serving as the porous wall of the invention. The pore-formers are nontoxic, and on their removal channels are formed that fill with fluid. The channels become a transport path for fluid. In a preferred embodiment, the non-toxic pore-forming agents are selected from the group consisting of water soluble inorganic and organic compounds and salts, carbohydrates, poly-alkylene glycols, poly($\alpha,\omega$) alkylenediols, esters of alkylene glycols, and glycols, that are used in a biological environment.

The microporous materials can be made by etched nuclear tracking, by cooling a solution of flowable polymer below the freezing point with subsequent evaporation of solvent to form pores, by gas formation in a polymer solution which upon curing results in pore formation, by cold or hot stretching at low or high temperatures until pores are formed, by leaching from a polymer a soluble component by an appropriate solvent, by ion exchange reaction, and by polyelectrolyte processes. Processes for preparing microporous materials are described in Synthetic Polymer Membranes: A Structural Perspective, 2nd Ed., by R. E. Kesting, Chapters 7 and 8, 1985, published by John Wiley & Sons, Inc.; Chemical Reviews, Ultrafiltration, Vol. 18, pages 373 to 455, 1934; Polymer Eng. and Sci., Vol. 11, No. 4, pages 284 to 288, 1971, J. Appl. Poly. Sci., Vol. 15, pages 811 to 829, 1971, and in U.S. Pat. Nos. 3,565,259; 3,615,024; 3,751,536; 3,801,692; 3,852,224; and 3,849,528.

It is generally desirable from a preparation standpoint to mix the polymer in a solvent. Exemplary solvents suitable for manufacturing the wall of the instant device include inert inorganic and organic solvents that do not adversely harm the core, wall, and the materials forming the final wall. The solvents broadly include members selected from the group consisting of aqueous solvents, alcohols, ketones, esters, ethers, aliphatic hydrocarbons, halogenated solvents, cycloaliphatic, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl ethyl ketone, methyl propyl ketone, n-hexane, ethyl lactate, n-heptane, ethylene glycol monoethyl ether, ethylene glycol monoethyl acetate, methylene dichloride, ethylene dichloride, propylene dichloride, carbon tetrachloride, nitroethane, nitropropane, tetrachloroethane, ethyl ether, isopropyl ether, cyclohexane, cyclooctane, dimethylbromamide, benzene, toluene, naphtha, 1,4-dioxane, tetrahydrofuran, diglyme, water, and mixtures thereof such as acetone and water, acetone and methanol, acetone and ethyl alcohol, methylene dichloride and methanol, and ethylene dichloride and methanol. Illustrative of mixed solvents are acetone-methanol (80:20), acetone-ethanol (90:10), methylene dichloride-methanol (80:20), ethyl acetate-ethanol (80:20), ethylene dichloride-methanol (80:20), methylene dichloride-methanol (50:50), methylene dichloride-methanol (78:22), acetone-water (90:10), chloroform-ethanol (80:20), methylene dichloride-ethanol (79:21), methylene chloride-methanol-water (15:10:1), carbontetrachloride-methanol (70:30), expressed as (weight:weight). Water-based latex forms of suitable polymeric materials are also within the scope of the invention.

Exemplary plasticizers suitable for the present purpose include plasticizers that lower the temperature of the second-order phase transition of the wall or the elastic modulus thereof, and also increase the workability of the wall and its flexibility. Plasticizers may increase or decrease the permeability of the wall to fluids including water and aqueous solutions. Plasticizers operable for the present purpose include both cyclic plasticizers and acyclic plasticizers. Typical plasticizers are those selected from the group consisting of phthalates, phosphates, citrates, adipates, tartrates, sebacates, succinates, glycolates, glycerolates, benzoates, myristates, polyethylene glycols, polypropylene glycols, and halogenated phenyls. Generally, from 0.001 to 50 parts of a plasticizer or a mixture of plasticizers are incorporated into 100 parts of wall forming material.

Exemplary plasticizers include dialkyl phthalates, dicycloalkyl phthalates, diaryl phthalates and mixed alkylaryl as represented by dimethyl phthalate, dipropyl phthalate, dioctyl phthalate, di-(2-ethyl-hexyl)-phthalate, diisopropyl phthalate, diamyl phthalate and dicapryl phthalate; alkyl and aryl phosphates such as triethyl phosphate, tributyl phosphate, trioctyl phosphate, tricresyl phosphate and triphenyl phosphate; alkyl citrate and citrate esters such as tributyl citrate, triethyl citrate, and acetyl triethyl citrate; alkyl adipates such as dioctyl adipate, diethyl adipate and di-(2-methyoxyethyl)-adipate; dialkyl tartrates such as diethyl tartrate and dibutyl tartrate; alkyl sebacates such as diethyl sebacate, dipropyl sebacate and dinonyl sebacate; alkyl succinates such as diethyl succinate and dibutyl succinate; alkyl glycolates, alkyl glycerolates, glycol esters and glycerol esters such as glycerol diacetate, glycerol triacetate, glycerol monolactate diacetate, methyl phthalyl ethyl glycolate, butyl phthalyl butyl glycolate, ethylene glycol diacetate, ethylene glycol dibutyrate, triethylene glycol dibutyrate and triethylene glycol dipropionate. Other plasticizers include polyethylene glycol 400, polyethylene glycol 20,000, camphor, N-ethyl-(o- and p-toluene) sulfonamide, chlorinated biphenyl, benzophenone, N-cyclohexyl-p-toluene sulfonamide, and substituted epoxides.

9

Suitable plasticizers can be selected for blending with the wall forming materials by selecting plasticizers that have a high degree of solvent power for the materials, are compatible with the materials over both the processing and use temperature range, exhibit permanence as seen by their tendency to remain in the plasticized wall, impart flexibility to the material and are non-toxic to animals, humans, avians, fishes and reptiles. Procedures for selecting a plasticizer having the described characteristics are disclosed in the Encyclopedia of Polymer Science and Technology, Vol. 10, pages 228 to 306, 1969, published by John Wiley & Sons, Inc. Also, a detailed description pertaining to the measurement of plasticizer properties including solvent parameters and compatibility such as the Hildebrand solubility parameter $\delta$, the Flory-Huggins interaction parameter $\chi$, and the cohesive-energy density, CED, parameters are disclosed in Plasticization and Plasticizer Processes, Advances in Chemistry Series 48, Chapter 1, pages 1 to 26, 1965, published by the American Chemical Society. The amount of plasticizer added generally is an amount sufficient to produce the desired wall and it will vary according to the plasticizer and the other wall forming materials. Usually about 0.001 part up to 50 parts of plasticizer can be used for 100 parts of wall material.

The expressions "flux regulator agent", "flux enhancing agent" and "flux decreasing agent" as used herein mean a compound that when added to a wall forming material assists in regulating the fluid permeability (flux) through the wall. The agent can be preselected to increase or decrease the fluid flux. Agents that produce a marked increase in permeability to a fluid such as water, are often essentially hydrophilic, while those that produce a marked decrease in permeability to fluids such as water, are often essentially hydrophobic. The flux regulators in some embodiments also can increase the flexibility and porosity of the lamina.

Examples of flux regulators include polyhydric alcohols and derivatives thereof, such as polyalkylene glycols of the formula H-(O-alkylene)$_n$-OH, wherein the bivalent alkylene radical is straight or branched chain and has from 1 to 10 carbon atoms and n is 1 to 500 or higher. Typical glycols include polyethylene glycols 300, 400, 600, 1500, 1540, 4000 and 6000 of the formula H-(OCH$_2$CH$_2$)$_n$-OH wherein n is typically 5 to 5.7, 8.2 to 9.1, 12.5 to 13.9, 29 to 36, 29.8 to 37, 68 to 84, and 158 to 204, respectively. Other polyglycols include the low molecular weight glycols of polypropylene, polybutylene and polyamylene.

Additional flux regulators include poly ($\alpha,\omega$) alkylenediols wherein the alkylene is straight or branched chain of from 2 to 10 carbon atoms such as poly(1,3)propanediol, poly(1,4) butanediol, poly(1,5)pentanediol and poly(l,6)hexanediol. The diols also include aliphatic diols of the formula HOC$_n$H$_{2n}$OH wherein n is from 2 to 10 and diols are optionally bonded to a non-terminal carbon atom such as 1,3-butylene glycol, 1,4-pentamethylene glycol, 1,5-hexamethylene glycol and 1,8-decamethylene glycol; and alkylenetriols having 3 to 6 carbon atoms such as glycerine, 1,2,3-butanetriol, 1,2,3-pentanetriol, 1,2,4-hexanetriol and 1,3,6-hexanetriol.

Other flux regulators include esters and polyesters of alkylene glycols of the formula HO-(alkylene-O)$_n$-H wherein the divalent alkylene radical includes the straight chain groups and the isomeric forms thereof having from 2 to 6 carbons and n is 1 to 14. The esters and polyesters are formed by reacting the glycol with either a monobasic or dibasic acid or anhydride. Exemplary flux regulators are ethylene glycol dipropionate, ethylene glycol butyrate, ethylene glycol diacetate, triethylene glycol diacetate, butylene glycol dipropionate, polyester of ethylene glycol with succinic acid, polyester of diethylene glycol with maleic acid, and polyester of triethylene glycol with adipic acid.

The amount of flux regulator added to a material generally is an amount sufficient to produce the desired permeability, and it will vary according to the lamina forming material and the flux regulator used to modulate the permeability. Usually from 0.001 parts up to 50 parts, or higher of flux regulator can be used to achieve the desired results.

Surfactants useful for the present coat forming purpose are those surfactants, when added to a wall forming material and other materials, aid in producing an integral composite that is useful for making the operative wall of a device. The surfactants act by regulating the surface energy of materials to improve their blending into the composite. The composite material is used for manufacturing devices that maintain their integrity in the environment of use during the agent release period. Generally, the surfactants are amphipathic molecules comprised of a hydrophobic part and a hydrophilic part. The surfactants can be anionic, cationic, nonionic or amphoteric. The anionic surfactants include sulfated, sulfonated, or carboxylated esters, amides, alcohols, ethers, aromatic hydrocarbons, aliphatic hydrocarbons, acylated amino acids and peptides. Metal alkyl phosphates are another class of anionic surfactant. Typically, cationic surfactants are primary, secondary, tertiary or quaternary alkylammonium salts, acylated polyamines, and salts of heterocyclic amines. Nonionic surfactants are typically esters and ethers of polyoxyalkylene glycols, polyhydric alcohols, or phenols. Poloxamers are included as nonionic surfactants. Surfactants are discussed in Surfactant Systems, Their Chemistry, Pharmacy, and Biology, D. Attwood and A. T. Florence, Chapman and Hall Pub. Co., 1983, pgs 1-8.

Examples of surfactants include potassium laurate, sodium dodecyl sulfate, hexadecylsulphonic acid, sodium dioctylsulphosuccinate, hexadecyl(cetyl)trimethylammonium bromide, dodecylpyridinium chloride, dodecylamine hydrochloride, N-dodecyl-N,N-dimethyl betaine, bile acids and salts, acacia, tragacanth, Igepal(™), sorbitane esters (Spans), polysorbates (Tweens), Triton-X(™) analogs, Brij(™) analogs, Myrj(™) analogs, pluronics, tetronics, surface active drug agents such as phenothiazines and tricyclic antidepressants.

Suitable surfactants can be selected from the above and from other surfactants for blending with wall forming materials by using the surfactant's hydrophile-lipophile balance number, HLB. This number represents the proportion between the weight percentages of hydrophilic and lipophilic groups in a surfactant. In use, the number indicates the behavior of the surfactant, that is, the higher the number the more hydrophilic the surfactant and the lower the number the more lipophilic the surfactant. The required HLB number for blending wall forming materials is determined by selecting a surfactant with a known HLB number, blending it with the materials and observing the results. A uniform composite is formed with the correct HLB number, while a non-uniform mixture indicates a different number is needed. This new number can be selected by using the prior HLB number as a guide. The HLB number is known to the art for many surfactants, and they can be experimentally determined. Generally a HLB number of 10 or less indicates lipophilic behavior and 10 or more indicates hydrophilic behavior. Also, HLB numbers are algebraically additive. Thus, by using a low number with a high number, blends of surfactant can be prepared having numbers intermediate between the two numbers. The concept of HLB is detailed in Remington's Pharmaceutical Sciences, 16th Ed., Mack Pub. Co., (1980), pages 316-319. The amount of surfactant needed is an amount that when blended with wall forming materials will form the desired wall composite, and it will vary according to the particular surfactant and materials that are blended to form the wall. Generally, the amount of surfactant will range from about 0.001 part up to 40 parts for 100 parts of wall.

The term drug includes any compound, or mixture thereof, that can be delivered from the device to produce a beneficial result. The term drug includes pesticides, herbicides, germicides, biocides, algicides, rodenticides, fungicides, insecticides, antioxidants, plant growth promoters, plant growth inhibitors, preservatives, disinfectants, sterilization agents, catalysts, chemical reactants, fermentation agents, foods, food supplements, nutrients, cosmetics, drugs, vitamins, sex sterilants, fertility inhibitors, fertility promoters, air purifiers, microorganism attenuators, and other agents that benefit the environment of use.

In the specification and the accompanying claims, the term "drug" includes any physiologically or pharmacologically active substances that produce a localized or systemic effect or effects in animals, which term includes mammals, humans and primates. The term also includes domestic household, sport or farm animals such as sheep, goats, cattle, horses and pigs, for administering to laboratory animals such as mice, rats and guinea pigs, and to fish to avians, to reptiles and zoo animals. The term "physiologically" as used herein denotes the administration of drug to produce desired levels and functions. The term "pharmacologically" denotes the study of the actions of drugs on living systems, including therapeutics, as defined in Dorland's Illustrated Medical Dictionary, 1974, published by W. B. Saunders Co., Philadelphia, PA. The phrase drug formulation as used herein means the drug is in the compartment by itself, or the drug is in the compartment mixed with an osmotic solute, binder, dye, solubility modulator, excipients and mixtures thereof. The active drug that can be delivered includes inorganic and organic compounds without limitation, including drugs that act on the peripheral nerves, adrenergic receptors, cholinergic receptors, nervous system, skeletal muscles, cardiovascular, smooth muscles, blood circulatory system, synaptic sites, neuroeffector junctional sites, endocrine and hormone systems, immunological system, reproductive system, skeletal system, autocoid systems, alimentary and excretory systems, inhibitory or autocoids and histamine systems, and those materials that act on the central nervous system such as hypnotics and sedatives.

Examples of beneficial drugs are disclosed in Remington's Pharmaceutical Sciences, 16th Ed., 1980, published by Mack Publishing Co., Eaton, Pa.; and in The Pharmacological Basis of Therapeutics, by Goodman and Gilman, 6th Ed., 1980, published by the MacMillan Company, London; and in The Merck Index, 10th Edition, 1983, published by Merck & Co., Rahway, N.J. The dissolved drug can be in various forms, such as charged molecules, charged molecular complexes or ionizable salts. Acceptable salts include, but are not limited to hydrochlorides, hydrobromide, sulfate, laurylate, palmitate, phosphate, nitrate, borate, acetate, maleate, tartrate, oleate, salicylate, ammonium, tromethamine, salts of metals, and amines or organic cations, for example quaternary ammonium.

Derivatives of drugs such as esters, ethers and amides which have ionization and solubility characteristics suitable for use herein can be used alone or mixed with other drugs. Also, a drug that is water insoluble can be used in a form that is a water soluble ionizable derivative thereof to effectively serve as a solute, and on its release from the device, is converted by enzymes, hydrolyzed by body pH or other metabolic processes to the original form, or to a biologically active form.

Specific examples of drugs that may be adapted for use include pentobarbital sodium, phenobarbital, secobarbital, thiopental and mixtures thereof; heterocyclic hypnotics such as dioxopiperidines and glutarimides; hypnotics and sedatives such as amides and ureas, exemplified by diethylisovaleramide and α-bromoisovaleryl urea; hypnotic and sedative urethanes and disulfanes; psychic energizers such as isocarboxazid, nialamide, phenelzine, imipramine, amitryptyline hydrochloride, tranylcypromine, pargylene, and protryptyline hydrochloride; tranquilizers such as chloropromazine, promazine, fluphenzaine, reserpine, deserpidine, and meprobamate; benzodiazepines such as diazepam and chlordiazepoxide; anticonvulsants such as primidone, phenytoin, and ethosuximide; muscle relaxants and antiparkinson agents such as mephenesin, methocarbomal, cyclobenzaprine hydrochloride, trihexylphenidyl hydrochloride, levodopa/carbidopa, and biperiden; antihypertensives such as α-methyldopa and the epivaloyloxyethyl ester of α-methyldopa; analgesics such as morphine sulfate, codeine sulfate, meperidine, and nalorphine; antipyretics and anti-inflammatory agents such as aspirin, indomethacin, sodium indomethacin trihydrate, salicylamide, naproxen, colchicine, fenoprofen, sulindac, diflunisal, diclofenac, indoprofen and sodium salicylamide; local anesthetics such as procaine, lidocaine, tetracaine and dibucaine; antispasmodics and muscle contractants such as atropine, scopolamine, methscopolamine, oxyphenonium, papaverine; prostaglandins such as $PGE_1$, $PGE_{2\alpha}$, $PGF_2$; antimicrobials and antiparasitic agents such as penicillin, tetracycline, oxytetracycline, chlorotetracycline, chloramphenicol, thiabendazole, ivermectin, and sulfonamides; antimalarials such as 4-aminoquinolines, 8-amino-quinolines and pyrimethamine; hormonal and steroidal agents such as dexamethasone, prednisolone, cortisone, cortisol and triamcinolone; androgenic steroids such as methyltestosterone; estrogenic steroids such as 17α-estradiol, α-estrodiol, estriol, α-estradiol 3-benzoate, and 17-ethynyl estradiol-3-methyl ether; progestational steroids such as progesterone; sympathomimetic drugs such as epinephrine, phenylpropanolamine hydrochloride, amphetamine, ephedrine and norepinephrine; hypotensive drugs such as hydralazine; cardiovascular drugs such as procainamide hydrochloride, amyl nitrite, nitroglycerin, dipyridamole, sodium nitrate and mannitol nitrate; diuretics such as chlorothiazide, acetazolamide, methazolamide, hydrochlorothiazide, amiloride hydrochloride and flumethiazide, sodium ethacrynate and furosemide; antiparasitics such as bephenium, hydroxynaphthoate, dichlorophen and dapsone; antineoplastics such as mechlorethamine, uracil mustard, 5-fluorouracil, 6-thioguanine and procarbazine; β-blockers such as pindolol, propranolol, metoprolol, oxprenolol, timolol maleate, atenolol; hypoglycemic drugs such as insulin, isophane insulin, protamine zinc insulin suspension, globin zinc insulin, extended insulin zinc suspension, tolbutamide, acetohexamide, tolazamide and chlorpropamide; antiulcer drugs such as cimetidine; nutritional agents such as ascorbic acid, niacin, nicotinamide, folic acid, choline, biotin, pantothenic acid; essential amino acids; essential fats; ophthalmic drugs such as timolol maleate, pilocarpine nitrate, pilocarpine hydrochloride, atropine sulfate, scopolamine; electrolytes such as calcium gluconate, calcium lactate, potassium chloride, potassium sulfate, sodium fluoride, ferrous lactate, ferrous gluconate, ferrous sulfate, ferrous fumurate and sodium lactate; and drugs that act on α-adrenergic receptors such as clonidine hydrochloride.

Additional preferred drugs include quinoline and naphthyridine carboxylic acids and related compounds, such as norfloxacin.

Additional preferred drugs include budesonide, enprofylline, tranilast, albuterol, theophylline, aminophylline, brompheniramine, chlorpheniramine, promethazine, diphenhydramine, azatadine, cyproheptadine, terbutaline, metaproterenol, and isoproterenol; drugs which are antidepressants such as doxepin, trazodone; antipsychotic drugs such as haloperidol, thioridazine, trifluoperazine; sedative hypnotic and antianxiety drugs such as triazolam, temazepam, chlorazepate, alprazolam, flurazepam, lorazepam, oxazepam, hydroxyzine, prazepam, meprobamate, butalbital, and chlorzoxazone; antiparkinson drugs such as benztropine and L-647,339; hormonal and steroidal drugs such as conjugated estrogens, diethylstilbesterol, hydroxy progesterone, medroxy progestrone, norethindrone, betamethasone, methylprednisolone, prednisone, thyroid hormone, and levothyroxine; antihypertensive and cardiovascular drugs such as isosorbide dinitrate, digoxin, nadolol, disopyramide, nifedipine, quinidine, lidocaine, diltiazem hydrochloride, verapamil, prazosin, captopril, enalapril, lisinopril, felodipine, tocainide, mexiletine, mecamylamine, and metyrosine; diuretic drugs such as spironolactone, chlorthalidone, metolazone, triamterene, methyclothiazide, and indacrinone; antiinflammatory drugs such as ibuprofen, ibuprofen lysinate, phenylbutazone, tolmetin, piroxicam, melclofenamate, auranofin, flurbiprofen and penicillamine; analgesic drugs such as acetaminophen, oxycodone, hydrocodone, and propoxyphene; antiinfective drugs such as cefoxitin, cefazolin, cefotaxime, cephalexin, nicarbazine, amprolium, ampicillin, amoxicillin, cefaclor, erythromycin, nitrofurantoin, minocycline, doxycycline, cefadroxil, miconazole, clotrimazole, phenazopyridine, clorsulon, fludalanine, pentizidone, cilastin, phosphonomycin, imipenem, arprinocid, and foscarnet; gastrointestinal drugs such as bethanechol, clidinium, dicyclomine, meclizine, prochlorperizine, trimethobenzamide, loperamide, ranitidine, diphenoxylate, famotidine, metoclopramide and omeprazole; anticoagulant drugs such as warfarin, phenindione, and anisindione; and other drugs such as trientine, cambendazole, ronidazole, rafoxinide, dactinomycin, asparaginase, nalorphine, rifamycin, carbamezepine, metaraminol bitartrate, allopurinol, probenecid, diethylpropion, dihydrogenated ergot alkaloids, nystatin, pentazocine, phenylpropanolamine, phenylephrine, pseudoephedrine, trimethoprim, lovastatin, pravastatin, simvastatin, and ivermectin.

The above list of drugs is not meant to be exhaustive. Many other drugs will certainly work in the instant invention.

The drug can be in the core compartment as a solution, dispersion, paste, cream, particle, granule, emulsion, suspension or powder. Also, the drug can be mixed with a binder, dispersant, emulsifier or wetting agent and dyes.

The core compartment containing the drug and the controlled release solubility modulator, as described herein, is typically in the form of a solid conventional tablet, pellet or particulate. The core can be comprised of a mixture of agents combined to give the desired manufacturing and delivery characteristics. The number of agents that may be combined to make the core is substantially without an upper limit with the lower limit equalling two components. It may be useful to buffer the core compartment to control the electrostatic charge of the drug.

The preferred specifications for the core are summarized below and include:

1. Core Drug Loading (size): 0.05 nanograms to 5 grams or more (includes dosage forms for humans and animals).

2. Osmotic pressure developed by a solution of the core: 8 to 500 atmospheres, typically, with commonly encountered water soluble drugs and excipients; however osmotic pressures greater than zero are within guidelines.

3. Core solubility: continuous, uniform release (zero-order kinetics) of 90% or greater of the initially loaded core mass is theoretically predicted if the ratio of the dissolvable core mass solubility, S, to the dissolvable core mass density, $\rho$ is S/$\rho$, is 0.1 or lower. Typically this occurs when 10% of the initially loaded dissolvable core mass saturates a volume of external fluid equal to the total volume of the initial dissolvable core mass.

S/$\rho$ ratios greater than 0.1 fall within the workings of the invention and result in lower percentages of initial core mass delivered under zero-order kinetics. S/$\rho$ can be selected to give acceptable combined characteristics of stability, release rate, and manufacturability.

4. Controlled Release Solubility Modulator: 0.01 to 75%, by weight, of the total core mass.

There is no critical upper limit as to the amount of drug that can be incorporated into a core mass and typically will follow the core loading (size) specification 1. The lower limit ratio of drug to excipient is dictated by the desired osmotic activity of the core composition, the desired time span and profile of release, and the pharmacological activity of the drug. Generally, the core will contain 0.01% to 90% by weight or higher, of an active agent in mixture with another solute(s). Representative of compositions of matter that can be released from the device and can function as a solute are, without limitation, those compositions soluble in fluids inside the core compartment as described.

The following examples illustrate the preparation of the drug-delivery devices of this invention and their controlled release of one or more therapeutically active ingredients into an enviroment of use.

## EXAMPLES

In the following examples diltiazem hydrochloride was used as the model drug.

The solubility of diltiazem hydrochloride is sensitive to the level of sodium chloride in the environment. For example, the solubilities of diltiazem hydrochloride in various aqueous sodium chloride concentrations were measured at 37°C.

| Approximate Solubility of Diltiazem HCl (mg/ml) | Sodium Chloride Concentration |
|---|---|
| <0.5 | saturated |
| 5 | 2 M |
| 10 | 1.5 M |
| 43 | 1.2 M |
| 185 | 1M |
| >500 | 0 |

13

The instant invention takes advantage of this dependence of diltiazem hydrochloride's solubility on the NaCl concentration to realize a drug-delivery system that delivers >80% of the drug load with zero-order kinetics. It should be noted that zero-order kinetics is the preferred release profile in the delivery of many drugs.

Example 1

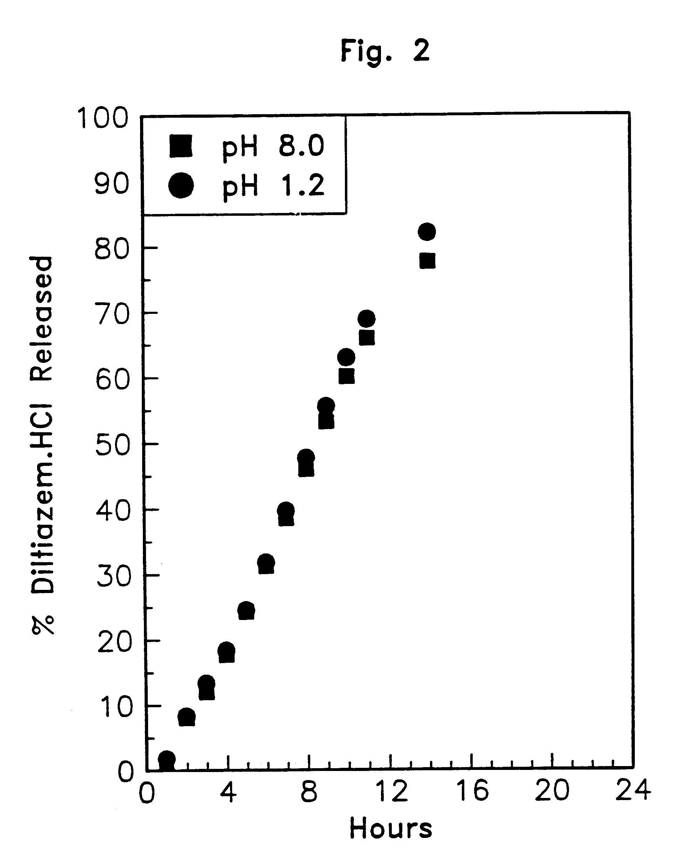

A plurality of drug-delivery systems containing controlled-release sodium chloride (C.R. NaCl) to modulate the solubility of diltiazem hydrochloride were prepared. First, the C.R. NaCl was made by taking a 700 g aliquot of sodium chloride crystals retained on a #30 sieve (600 $\mu$m opening) and applying a microporous wall to these crystals by standard fluidized-bed spray coating techniques. The spray solution was 100 g cellulose acetate propionate (48% by weight propionyl) dissolved in a 3:1 dichloromethane/methanol solvent blend. To this was added 4 g triacetin as a rate modifier influencing the release of NaCl. This solution was sprayed onto the NaCl crystals in a commercial Uni-Glatt fluidized bed coating machine. The sodium chloride crystals were coated to give 8 hours zero-order NaCl release with 2-3 hours additional first-order release when placed into 37°C water. The release of NaCl was analysed with a Jenway PCM3 conductivity meter. Next, a wet granulation was made containing diltiazem hydrochloride, adipic acid, and citric acid mixed 40:11:8 with 4% w/w polyvinylpyrrolidone (29-32K) added as a binder. It was calculated that 45.6 mg of NaCl (95 mg total weight C.R. NaCl including coat) would be needed to maintain the NaCl concentration at approximately 1M within the 0.25 ml core compartment throughout a typical 8-14 hour delivery period. 123 mg aliquots of the dried granules were mixed with 95 mg C.R. NaCl and 40 mg uncoated sodium chloride. The uncoated NaCl was added to the core to rapidly suppress the solubility of diltiazem hydrochloride prior to the steady-state release of the C.R. NaCl. This mixture of granules was formed into core tablets by compressing 258 mg aliquots (80 mg drug load) into a 0.67 cm (1/4") standard concave tabletting die by applying a 1 ton force with a single station hydraulic press. Next, a microporous coat was applied to these cores. 72 g cellulose acetate having an acetyl content of 39% was dissolved in a dichloromethane/methanol solvent blend. To this was added 54 g sorbitol as pore former dissolved in a water/methanol solvent blend. The composite solution contained water:methanol:dichloromethane in an approximate 1:10:15 ratio. This solution was sprayed onto the cores in a commercial Uni-Glatt fluidized-bed coating machine. A wall 400 $\mu$m thick was applied to the tablet cores. The diltiazem hydrochloride release from these devices in vitro into 900 ml volumes of 37°C, pH 1.2 HCl buffer and pH 8.0 phosphate buffer, both made isotonic with NaCl, was monitored in a USP Dissolution Method #2 apparatus with constant stirring at 50 rpm. HPLC was used to assay for diltiazem. The release of diltiazem HCl at both pH's is shown in Fig. 2. The release profile of diltiazem from these devices shows greater than 80% of the drug released with zero-order kinetics.

Example 2

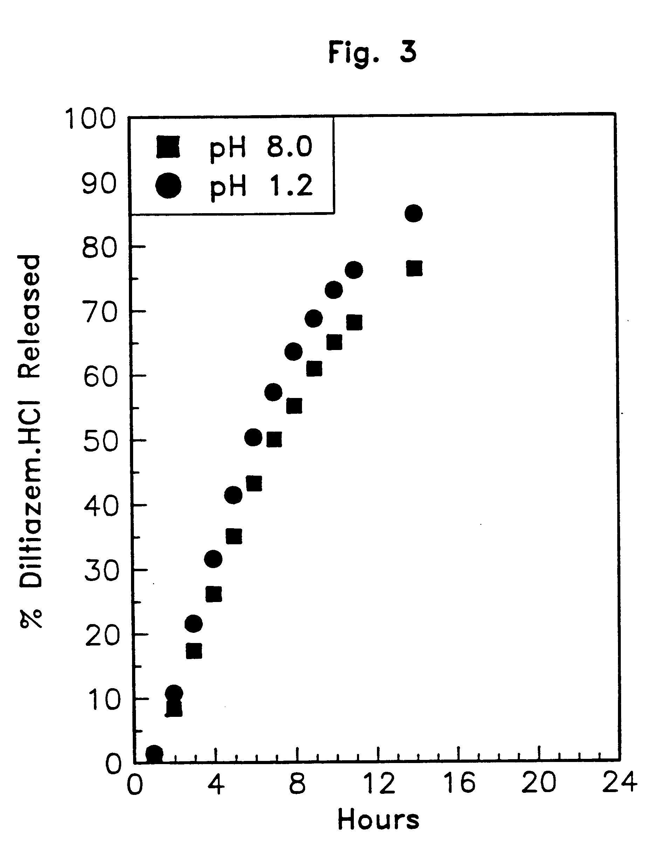

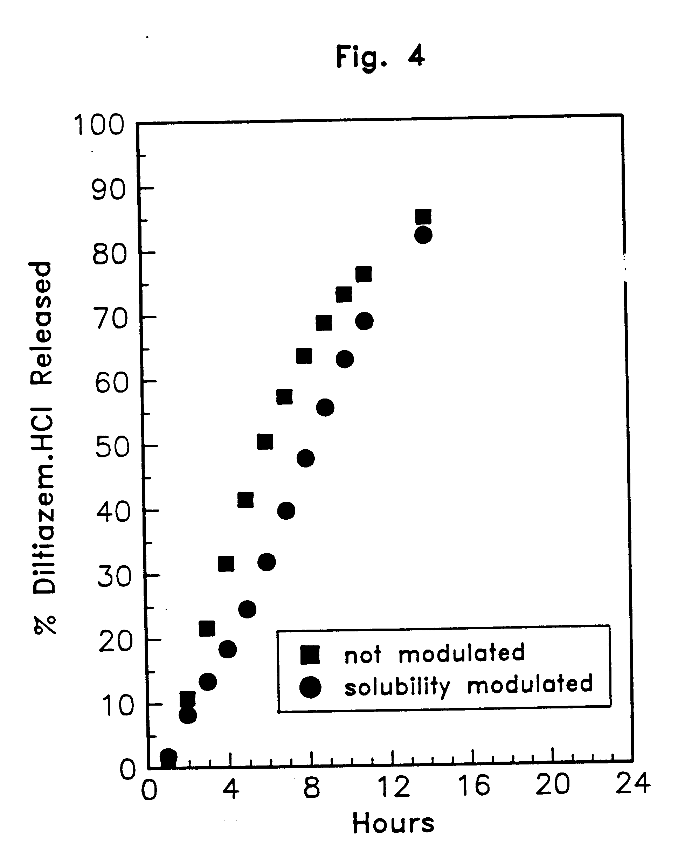

A plurality of drug-delivery devices were prepared that did not contain either C.R. NaCl or rapid release NaCl. A wet granulation was made containing diltiazem hydrochloride, adipic acid, and citric acid mixed 40:11:8 with 4% w/w polyvinylpyrrolidone (29-32K) added as a binder. 123 mg aliquots (80 mg drug load) of the dried granules were compressed into a 0.64 cm (1/4") standard concave tabletting die applying a 1 ton force with a single station hydraulic press. Next, a microporous coat identical in composition to the coat applied to the cores in example 1 was applied to these cores. Again, a wall thickness of 400 $\mu$m was applied. The diltiazem hydrochloride release from these devices was monitored as in Example 1. The release of diltiazem HCl is shown in Fig. 3. The release profile of diltiazem from these devices shows less than 50% of the drug release with zero-order kinetics. After 5 hours of release the profile becomes first-order in nature deviating markedly from the preferred zero-order release. Fig. 4 shows a comparison of the average release profiles of Example 1 and Example 2 in pH 1.2 buffer. Examining first the release profile of the devices prepared in Example 2 (no NaCl) it is seen that greater than 50% of the drug load is released in the first 6 hours. This corresponds to a release rate of 6.67 mg/hour. During the next 8 hours only 34% of the drug load is released. This corresponds to an average release rate of 3.5 mg/hour during hours 6-14. This represents a 48% decrease in the rate of drug delivery after 6 hours. If the therapeutic index of the drug is small, this type of first-order release would not be an effective pattern of drug delivery. This deviation from zero-order release seen with the Example 2 devices (Fig. 3) is what those skilled in the art of drug-delivery attempt to obviate. In contrast, the devices in Example 1 (Fig. 2) released approximately 31% of the drug load in the first 6 hours. This corresponds to a release rate of 4.2 mg/hour. During the next 8 hours 50% of the drug load was released corresponding to a release rate of 4.9 mg/hr. This zero-order release performance for >80% of the initial drug load through use of C.R. NaCl represents a dramatic improvement over the performance when NaCl was not used to modulate the release.

It is a purpose of the instant invention to realize a system that will optimize the zero-order kinetic performance that prior art drug-delivery devices can not achieve.

Example 3

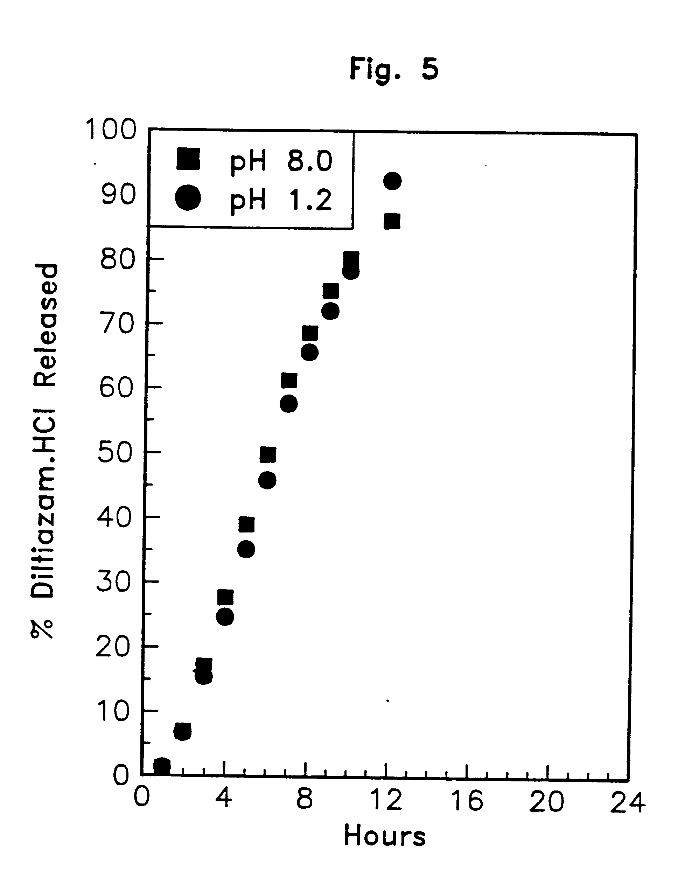

A plurality of drug-delivery devices containing C.R. NaCl were prepared. A wet granulation containing 58% w/w diltiazem hydrochloride, 18% w/w adipic acid, 13% w/w citric acid, 7.2% sodium chloride, and 4% w/w polyvinylpyrrolidone (29-32K) used as a binder was prepared. 130 mg aliquots of the dried granules were mixed with 95 mg C.R. NaCl (prepared in Example 1) and 40 mg uncoated NaCl. The composite mixture weighing 265 mg was compressed into a core compartment using a 0.64 cm (1/4") standard concave tabletting die applying a 1 ton force with a single station hydraulic press. Next, a microporous coat was applied to these tablet cores. 54 g cellulose acetate having an acetyl content of 39% and 18 g cellulose acetate having an acetyl content of 32% were dissolved in a dichloromethane/methanol solvent blend. To this was added 54 g sorbitol as pore former and 14.4 g polyethylene glycol 400 as a flux enhancer/plasticizer in a water/methanol solvent blend. The composite solution contained water:methanol:dichloromethane in an apoproximate 1:10:15 ratio. This solution was sprayed onto the cores in a commercial Uni-Glatt fluidized bed coating machine. A wall 400 $\mu$m thick was applied. The diltiazem hydrochloride release from these devices was monitored as in Example 1 with the release of diltiazem HCl shown in Fig. 5. Again, the kinetics of diltiazem release are zero-order for greater than 80% of the drug load.

Example 4

A plurality of drug-delivery systems containing controlled-release sodium chloride (C.R. NaCl) to modulate the solubility of diltiazem hydrochloride are prepared. First, the C.R. NaCl is made by taking a 700 g aliquot of sodium chloride crystals retained on a #30 sieve (600 $\mu$m opening) and applying a microporous wall to these crystals by standard fluidized-bed spray coating techniques. The spray solution is 100 g cellulose acetate propionate (48% by weight propionyl) dissolved in a 3:1 dichloromethane/methanol solvent blend. To this is added 4 g triacetin as a rate modifier influencing the release of NaCl. This solution is sprayed onto the NaCl crystals in a commercial Uni-Glatt fluidized bed coating machine. The sodium chloride crystals are coated to give 8 hours zero-order NaCl release with 2-3 hours additional first-order release when placed into 37°C water. The release of NaCl is analysed with a Jenway PCM3 conductivity meter. Next, a wet granulation is made containing diltiazem hydrochloride, adipic acid, and citric acid mixed 40:11:8 with 4% w/w polyvinylpyrrolidone (29-32K) added as a binder. It was calculated that 45.6 mg of NaCl (95 mg total weight C.R. NaCl including coat) would be needed to maintain the NaCl concentration at approximately 1M within the 0.25 ml core compartment throughout a typical 8-14 hour delivery period. 123 mg aliquots of the dried granules are mixed with 95 mg C.R. NaCl and 40 mg uncoated sodium chloride. The uncoated NaCl is added to the core to rapidly suppress the solubility of diltiazem hydrochloride prior to the steady-state release of the C.R. NaCl. This mixture of granules is formed into core tablets by compressing 258 mg aliquots (80 mg drug load) into a 0.64 cm (1/4") standard concave tabletting die by applying a 1 ton force with a single station hydraulic press. Next, a semipermeable coat is applied to these cores. 72 g cellulose acetate having an acetyl content of 39% was dissolved in a dichloromethane/methanol solvent blend. The composite solution contained water:methanol:dichloromethane in an approximate 1:10:15 ratio. This solution is sprayed onto the cores in a commercial Uni-Glatt fluidized-bed coating machine. A wall 100 to 200 $\mu$m thick is applied to the tablet cores and a hole 0.15 mm in diameter is drilled through the wall. The diltiazem hydrochloride release from these devices in vitro into 900 ml volumes of 37°C, pH 1.2 HCl buffer and pH 8.0 phosphate buffer, both made isotonic with NaCl, can be monitored in a USP Dissolution Method #2 apparatus with constant stirring at 50 rpm. HPLC can be used to assay for diltiazem.

EXAMPLE 5

A plurality of drug-delivery devices containing C.R. NaCl are prepared. A wet granulation containing 58% w/w diltiazem hydrochloride, 18% w/w adipic acid, 13% w/w citric acid, 7.2% sodium chloride, and 4% w/w polyvinylpyrrolidone (29-32K) used as a binder is prepared. 130 mg aliquots of the dried granules are mixed with 95 mg C.R. NaCl (prepared in Example 4) and 40 mg uncoated NaCl. The composite mixture weighing 265 mg is compressed into a core compartment using a 0.64 cm (1/4") standard concave

EP 0 314 206 B1

tabletting die applying a 1 ton force with a single station hydraulic press. Next, a semi-permeable coat is applied to these tablet cores. 54 g cellulose acetate having an acetyl content of 39% and 18 g cellulose acetate having an acetyl content of 32% is dissolved in a dichloromethane/methanol solvent blend. To this is added 14.4 g polyethylene glycol 400 as a flux enhancer/plasticizer in a water/methanol solvent blend. The composite solution contains water:methanol:dichloromethane in an apoproximate 1:10:15 ratio. This solution is sprayed onto the cores in a commercial Uni-Glatt fluidized bed coating machine. A wall 100 to 200 $\mu$m thick is applied and a hole 0.15 mm in diameter is drilled through the wall. The diltiazem hydrochloride release from these devices is monitored as in Example 4.

EXAMPLE 6

A plurality of drug-delivery devices containing C.R. NaCl are prepared. A wet granulation containing 58% w/w diltiazem hydrochloride, 18% w/w adipic acid, 13% w/w citric acid, 7.2% sodium chloride, and 4% w/w polyvinylpyrrolidone (29-32K) used as a binder is prepared. 130 mg aliquots of the dried granules are mixed with 95 mg C.R. NaCl (prepared in Example 4) and 40 mg uncoated NaCl. The composite mixture weighing 265 mg is compressed into a core compartment using a 0.64 cm (1/4") standard concave tabletting die applying a 1 ton force with a single station hydraulic press as in Example 6.

The cores are coated with a semipermeable wall 200 microns thick containing a drilled 0.15 mm diameter hole as described in Example 4. The devices are then spray coated with a 110 micron thick layer of a water soluble mixture of polyvinyl pyrrolidone and sorbitol mixed in a 1:25 weight ratio. This layer is then covered by a microporous wall 100 $\mu$m thick by spray coating a dichloromethane/methanol/water solution of a 1:1:1 blend of cellulose acetate having an acetyl content of 32%, cellulose acetate having an acetyl content of 39%, and sorbitol. The sorbitol is incorporated as a pore forming additive.

**Claims**

1. An osmotic drug delivery device for the controlled release of a therapeutically active ingredient into an environment of use which comprises:
    (A) a core composition comprising
        (a) a plurality of controlled release solubility modulating units which modulate and increase solubility of said therapeutically active ingredient within said core comprising solubility modulating agents each of which is either a water soluble salt, a complexing agent or a surfactant and which is surrounded by a water insoluble coat containing at least one pore forming additive dispersed throughout said coat;
        (b) a therapeutically active ingredient; and
    (B) a water insoluble microporous wall surrounding said core composition and prepared from
        (i) a polymer material that is permeable to water but substantially impermeable to solute and
        (ii) 0.1 to 75% by weight, based on the total weight of (i) and (ii), of at least one water leachable pore forming additive dispersed throughout said wall.

2. A drug-delivery device according to Claim 1, wherein the solubility modulating agent is a complexing agent selected from cyclodextrins, polyethylene glycols, polyvinyl-pyrrolidone, sodium carboxymethylcellulose, tetracycline derivatives, caffeine, picric acid, quinhydrone, hydroquinone, and bile salts and acids.

3. A drug-delivery device according to Claim 1, wherein the solubility modulating agent is a surfactant selected from potassium laurate, sodium dodecyl sulfate, hexadecylsulphonic acid, sodium dioctylsulphosuccinate, hexadecyl(cetyl)trimethylammonium bromide, dodecylpyridinium chloride, dodecylamine hydrochloride, N-dodecyl-N,N-dimethyl betaine, bile acids and salts, acacia, tragacanth, polyethylene glycol nonylphenyl ether polymers (Igepal (™)), sorbitan esters, polysorbates, 2-methyl-2-[(1-oxo-2-propenyl)amino]-2 propenoic acid polymers (Triton-X (™) analogues), $\alpha$-hydroxy-omega-cetyl-poly-(ethylene oxide)s (Brij (™) analogues), poly(oxyethylene) stearates (Myrj (™) analogues), pluronics, tetronics, phenothiazines and tricyclic antidepressants.

4. A drug-delivery device according to Claim 1, wherein the therapeutically active ingredient is soluble in an external fluid and exhibits an osmotic pressure gradient across the wall against the external fluid.

16

**5.** A drug-delivery device according to Claim 1, wherein the therapeutically active ingredient has limited solubility in the external fluid and is mixed with an osmotically effective solute that is soluble in the fluid, which exhibit an osmotic pressure gradient across the wall against the external fluid.

**Patentansprüche**

**1.** Osmotische Arzneimittel-Abgabevorrichtung zur kontrollierten Freisetzung eines therapeutisch aktiven Bestandteils in eine Anwendungsumgebung, umfassend

(A) eine Kernmasse, umfassend

(a) eine Vielzahl von löslichkeitsregulierenden Einheiten zur kontrollierten Freisetzung, die die löslichkeit des genannten therapeutisch aktiven Bestandteiles innerhalb des genannten Kernes, der die löslichkeitsregulierenden Mittel umfaßt, regulieren und vergrößern, wobei jedes davon entweder ein wasserlösliches Salz, ein Komplexierungsmittel oder ein oberflächenaktives Mittel darstellt, und der umgeben ist von einem wasserunlöslichen Überzug, der mindestens einen porenbildenden Zusatzstoff enthält, der in dem genannten Überzug dispergiert ist;

(b) einen therapeutisch aktiven Bestandteil; und

(B) eine wasserunlösliche mikroporöse Wand, die die genannte Kernmasse umgibt und hergestellt ist aus

(i) polymerem Material, das für Wasser durchlässig ist, jedoch für gelösten Stoff im wesentlichen undurchlässig ist und

(ii) 0,1 bis 75 Gew.-%, bezogen auf das Gesamtgewicht von (i) und(ii), von mindestens einem wasserdurchlässigen porenbildenden Zusatzstoff, der in der genannten Wand dispergiert ist.

**2.** Arzneimittel-Abgabevorrichtung nach Anspruch 1, bei der das Löslichkeitsregulierungsmittel ein Komplexierungsmittel ist, ausgewählt aus Cyclodextrinen, Polyethylenglycolen, Polyvinylpyrrolidon, Natriumcarboxymethylcellulose, Tetracyclinderivaten, Koffein, Pikrinsäure, Quinhydron, Hydroquinon und Gallensalzen und -säuren.

**3.** Arzneimittel-Abgabevorrichtung nach Anspruch 1, bei der das Löslichkeitsregulierungsmittel ein oberflächenaktives Mittel ist, ausgewählt aus Kaliumlaurat, Natriumdodecylsulfat, Hexadecylsulphonsäure, Natriumdioctylsulphonsuccinat, Hexadecyl-(cetyl)-trimethylammoniumbromid, Dodecylpyridiniumchlorid, Dodecylaminhydrochlorid, N-dodecyl-N,N-dimethylbetain, Gallensäuren und -salzen, Acacia, Tragacanth, Polyethylenglycol-Nonylphenylether-Polymeren (Igepal ($^{TM}$)), Sorbitanester, Polysorbaten, 2-Methyl-2-[(1-oxo-2-propenyl)-amino]-2-propensäure-Polymeren (Triton-X($^{TM}$)-Analoga), α-Hxdroxy-omega-cetyl-poly(ethylenoxid(en)) (Brij ($^{TM}$)-Analoga), Poly(oxyethylen)-stearaten (Myrj ($^{TM}$)-Analoga), Pluronen, Tetronen, Phenothiazinen und tricyclischen Antidepressiva.

**4.** Arzneimittel-Abgabevorrichtung nach Anspruch 1, bei der der therapeutisch aktive Bestandteil in einer externen Flüssigkeit löslich ist und einen osmotischen Druckgradienten quer zur Wand gegen die externe Flüssigkeit aufweist.

**5.** Arzneimittel-Abgabevorrichtung nach Anspruch 1, bei der der therapeutisch aktive Bestandteil eine begrenzte Löslichkeit in der externen Flüssigkeit aufweist und mit einem osmotisch wirksamen gelösten Stoff vermischt ist, der in der Flüssigkeit löslich ist, die einen osmotischen Druckgradienten quer zur Wand gegen die externe Flüssigkeit aufweist.

**Revendications**

**1.** Dispositif osmotique de distribution de médicaments destiné à la libération contrôlée d'un ingrédient thérapeutiquement actif dans un environnement d'utilisation, qui comprend :

(A) une composition de coeur comprenant (a) une série d'unités de modulation de solubilité à libération contrôlée qui modulent et augmentent la solubilité dudit ingrédient thérapeutiquement actif au sein dudit coeur comprenant des agents de modulation de solubilité dont chacun est ou bien un sel hydrosoluble, ou un agent complexant ou un tensioactif et qui est entouré d'une couche non hydrosoluble contenant au moins un additif porogène dispersé dans ledit enrobage ; (b) un ingrédient thérapeutiquement actif ; et

(B) une paroi microporeuse non hydrosoluble entourant ladite composition de coeur et préparée à partir de (i) une matière polymère qui est perméable à l'eau, mais sensiblement imperméable au soluté, et (ii) de 0,1 à 75% en poids, par rapport au poids total de (i) et (ii), d'au moins un additif porogène lixiviable par l'eau dispersé dans la totalité de ladite paroi.

2. Dispositif de distribution de médicaments selon la revendication 1, dans lequel l'agent modulant la solubilité est un agent complexant choisi parmi les cyclodextrines, les polyéthylène-glycols, la polyvinylpyrrolidone, la carboxyméthylcellulose sodique, les dérivés de tétracycline, la caféine, l'acide picrique, la quinohydrone, l'hydroquinone et les sels et les acides biliaires.

3. Dispositif de distribution de médicaments selon la revendication 1, dans lequel l'agent modulateur de solubilité est un tensioactif choisi parmi le laurate de potassium, le dodécylsulfate de sodium, l'acide hexadécylsulfonique, le dioctylsulfosuccinate de sodium, le bromure d'hexadécyl(cétyl)-triméthylammonium, le chrorure de dodécylpyridinium, le chlorhydrate de dodécylamine, la N-dodécyl-N,N-diméthylbétaïne, les acides et les sels biliaires, l'acacia, l'adragante, les polymères d'éther nonylphénylique de polyéthylène-glycol (Igepal, marque déposée), les esters de sorbitanne, les polysorbiates, les polymères d'acide 2-méthyl-2-((1-oxo-2-propényl)amino)-2-propénoïque (analogues de Triton X, marque déposée), le (ou les) alpha-hydroxy-oméga-céthyl-poly(oxyde d'éthylène) (analogues de Brij, marque déposée), les stéarates de poly(oxyéthylène) (analogues de Myrj, marque déposée), les pluronics, les tétronics, les phénothiazines et les antidépresseurs tricycliques.

4. Dispositif de distribution de médicaments selon la revendication 1, dans lequel l'ingrédient thérapeutiquement actif est soluble dans un fluide externe et fait preuve d'un gradient de pression osmotique à travers la paroi contre le fluide externe.

5. Dispositif de distribution de médicaments selon la revendication 1, dans lequel l'ingrédient thérapeutiquement actif possède une solubilité limitée dans le fluide externe et il est mélangé avec un soluté osmotiquement efficace qui est soluble dans le fluide et qui possède un gradient de pression osmotique à travers la paroi contre le fluide externe.

FIG. 1

FIG. 1a

## Fig. 2

## Fig. 3

# Fig. 4

Fig. 5

FIG. 6

FIG. 6a

FIG. 6b